# EUROPÄISCHE PATENTSCHRIFT

(11) **EP 1 416 987 B1**
(45) Veröffentlichungstag und Bekanntmachung des Hinweises auf die Patenterteilung: **09.02.2011**
(21) Anmeldenummer: 02758116.4
(22) Anmeldetag: 24.07.2002
(51) Int. Cl.: A61M 5/168

(54) **VORRICHTUNG ZUM SELBSTDOSIEREN UND ZUR STEUERUNG DER DOSIERUNG EINES FLÜSSIGEN MEDIKAMENTS**
DEVICES FOR SELF-DOSING A LIQUID MEDICAMENT AND FOR CONTROLLING THE DOSAGE OF THE SAME
DISPOSITIFS D'AUTODOSAGE ET DE REGULATION DU DOSAGE D'UN MEDICAMENT LIQUIDE

(30) Priorität: 26.07.2001 DE 10136081
(43) Veröffentlichungstag der Anmeldung: 12.05.2004
(73) Patentinhaber: Rahe-Meyer, Niels, 30177 Hannover (DE)
(72) Erfinder: Rahe-Meyer, Niels, 30177 Hannover (DE)
(74) Vertreter: Chambosse, Hans-Joachim
(86) Internationale Anmeldenummer: PCT/DE2002/002716
(87) Internationale Veröffentlichungsnummer: WO 2003/011375

(56) Entgegenhaltungen:
- WO-A-90/07353
- WO-A-91/12834
- DE-A- 1 541 363
- US-A- 4 585 442

## Beschreibung

Die Erfindung betrifft eine Vorrichtung zur Dosierung und zur Steuerung und Kontrolle der Dosierung von das Gefäßsystem, vorzugsweise intravenös, verabreichten Medikamenten. Dosiergerät Vorrichtungen, mit denen der Zeitpunkt und in Grenzen auch die Dosierung der Menge des zu verabreichenden Medikaments durch Nichtmediziner und namentlich durch den Patienten selbst vorgenommen werden können, sind an sich bekannt. Die bekannten Vorrichtungen zur patientenkontrollierten Dosierung sind jedoch auf aufwendige Präzisionspumpen und für die einzelne Vorrichtung auf spezifisches steriles Einmalmaterial wie Schlauchsysteme und/oder Medikamentenreservoirs angewiesen. Die Medikamentendosis und -konzentration muß bei der Medikamentenbeschickung und der Grundeinstellung der Vorrichtung auf die Kapazität des jeweiligen speziellen Medikamentenbehälters umgerechnet werden, was eine ausführliche Einweisung und Schulung an Umrechnungstabellen für die Geräte erforderlich macht. Die hohen Anschaffungskosten für die bekannten Vorrichtungen, die hohen Kosten für spezielles Einmalmaterial und der Personalaufwand für die Beschickungen und Einstellungen der Geräte durch den speziell ausgebildeten Arzt sind derart hoch, daß sie gerade auf dem Gebiet der Schmerztherapie, insbesondere Akutschmerztherapie bei sich verändernden chronischen Schmerzen, weit geringer eingesetzt werden können, als es von der Sache her anzustreben ist.

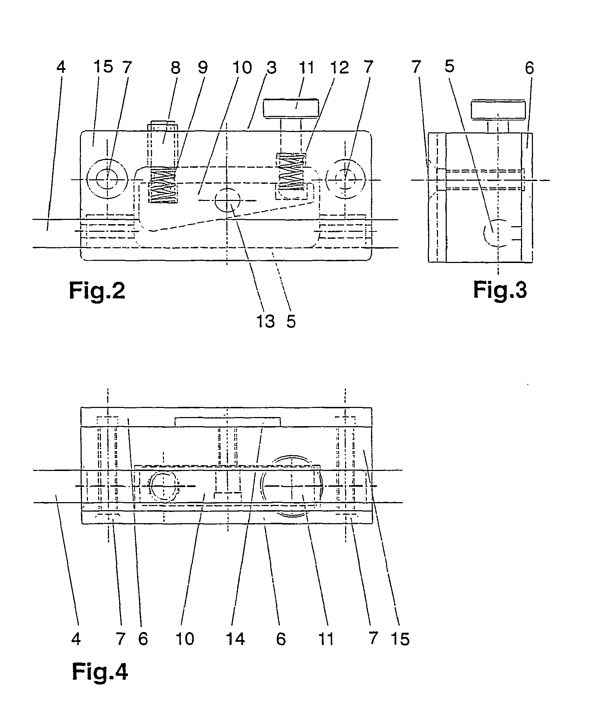

Aus der DE-A- 1541363 ist eine Vorrichtung zur Regulierung der Tropfgeschwindigkeit einer Schwerkraftinfusion bekannt, mit der die Tropfenfrequenz der Infusion genau eingestellt und konstant gehalten werden soll. Dies soll mittels einer in das Infusionssystem eingeschalteten Einstellkammer eines mechanisch verstellbaren Druckstücks und einer elektromagnetisch angetriebenen Sperrung, die mittels einer Nockenscheibe und einer Übersetzung den Infusionsschlauch durch periodische Bewegungen periodisch sperrt und wieder freigibt, erreicht werden. Dabei handelt es sich um einen technisch und kostenmäßig aufwendigen Mechanismus, der in der Handhabung zudem kompliziert ist und mit der Einstellkammer (Abb. 3 bis 5) unabdingbar spezifisches steriles Material benötigt. Das Dosiergerät eignet sich schon deshalb in keiner Weise als Vorrichtung zum Selbstdosieren und zur Steuerung der Dosierung eines flüssigen Medikaments durch den Patienten selbst oder einen Pfleger ohne spezielle medizinisch-technische Ausbildung.

Dokument US 45 85 442 zeigt den nächstkommenden Stand der Technik und offenbart die Merkmale der Oberbegriffe der Ansprüche 1, 3 und 6.

Aus der EP 0 504 255 B1 ist eine selbstgesteuerte Pumpvorrichtung, nämlich eine Vorrichtung zur patientengesteuerten Abgabe eines Heilmittels bekannt, die ein Dosisreservoir für das Medikament mit einer Pumpeinrichtung aus elastischem Material aufweist, wobei letztere vom Patienten betätigt wird und das Medikament durch die Pumpwirkung über einen Schlauch und einen angeschlossenen intravenösen Katheter dem Körper zuführt. Das flüssige Medikament wird aus einem gesonderten Vorratsbehälter in das Dosisreservoir eingezogen, indem die Pumpeinrichtung in die Ausgangsform zurückkehrt, wenn sie nicht mehr niedergedrückt wird und dabei Unterdruck entsteht. Diese Pumpvorrichtung hat neben dem Erfordernis einer Mehrzahl getrennter Bauteile den maßgeblichen Nachteil, daß die Pumpeinrichtung keine zuverlässige Dosierung der Menge des Medikaments und der Zeitdauer der Verabreichung zuläßt, weil die Andruckskraft und die Betätigungszeit völlig vom willkürlichen Verhalten des Patienten oder sonstigen Bedieners der Vorrichtung abhängen. Außerdem besteht ohne Einsatz eines Rückschlagventils am Ausgang der Pumpvorrichtung zum distalen Ende die Gefahr, daß nach dem Loslassen der Pumpeinrichtung diese flüssiges Medikament auch aus der Zuleitung zum Patienten zurückzieht. Die vorgeschlagene ergänzende Vorrichtung zur zeitlichen Festlegung der Inbetriebsetzung der Pumpeinrichtung unabhängig von der Betätigung durch den Patienten ist zum einen konstruktiv aufwendig und hebt umgekehrt die Möglichkeit der individuellen Steuerung der Pumpvorrichtung durch den Patienten weitgehend auf.

Aus der EP 0 592 483 B1 ist ebenfalls eine vom Patienten gesteuerte Infusionsvorrichtung bekannt, die eine Verdrängerpumpe mit einem bestimmten Arbeitsvolumen und einen getrennt davon angeordneten Speicher für das Arzneimittel aufweist, wobei jeweils die Pumpe zunächst über eine Verbindungsleitung durch Unterdruck mit der Arzneimitteldosis gefüllt werden muß und dann durch Betätigung der Pumpe das Arzneimittel über weitere Leitungen dem Patienten über ein distales Ende zugeführt wird. Dabei ist zusätzlich zu den getrennten Bauteilen ein Einwegventil (Rückschlagventil) unabdingbar, um bei der Füllung der Pumpe aus dem Speicher einen Rückzug von Arzneimittel durch den Unterdruck aus der Zuführungsleitung zum Patienten zu verhindern. Darüber hinaus wird die Menge des zu verabreichenden Medikaments in der Pumpe maßgeblich durch den Innendurchmesser der Verbindungsleitung vom Speicher zur Pumpe festgelegt und damit die Variabilität der Verabreichung des flüssigen Medikaments maßgeblich eingeschränkt. Zudem ist bei dieser vorbekannten Vorrichtung als deren eigentlicher Erfindungsgegenstand eine demontierbare Verbindung zwischen der Pumpe und der zum Patienten führenden Leitung vorgesehen, um eine beschleunigte Zuführung des Medikaments zu ermöglichen.

In der EP 0 523 456 B1 ist ein Gerät und System zum Selbstdosieren eines flüssigen Medikaments beschrieben, bei denen gleichfalls eine Pumpe, bestehend aus einer Kammer zur Aufnahme der flüssigen Medizin und einem vom Patienten niederzudrückenden Druckknopf, mit einem gesonderten Medizinbeutel über einen dünnen langen Schlauch verbunden ist. Gegenstand der Erfindung nach dieser Druckschrift ist die Anordnung der mit dem Druckknopf zusammenwirkenden Spiralfeder an der Außenseite der Medizinkammer, um die Höhe der Vorrichtung zu vermindern. Auch bei dieser Vorrichtung wird ein Einweg-Rückschlagventil im Anschluß an den Pumpenausgang in der Schlauchzuführung zum Patienten benötigt. Außerdem wird die Dosierung bzw. Strömungsrate der flüssigen Medizin per se durch den Innendurchmesser des Verbindungsschlauchs vom Medizinbeutel zur Pumpe bestimmt einschließlich der Wiederfüllzeit der Pumpenkammer, so daß die Menge der zu verabreichenden Medizin und der Zeitabstand, namentlich auch bei Bolusgaben nicht oder jedenfalls nur aufwendig und mit erheblichem Zeitaufwand zu ändern sind.

Aufgabe der vorliegenden Erfindung ist es daher, eine Vorrichtung zur Dosierung und zur Steuerung und Kontrolle der Dosierung von in das Gefäßsystem, vorzugsweise intravenös, zu verabreichenden flüssigen Medikamenten zu schaffen, die die bekannten Vorrichtungen so vereinfacht, daß die Anwendung durch Patienten und/oder Pflegepersonal ohne spezielle medizinische Ausbildung möglich ist und die Anwendung des Verfahrens durch vereinfachte Vorrichtungen kostenmäßig erheblich verbilligt wird.

Diese Aufgabe wird im wesentlichen durch die Merkmale der Patentansprüche 1, 3 und 6 gelöst. Ausführungsformen der Vorrichtungen sind Gegenstand der weiteren Patentansprüche.

Nachfolgend werden die Vorrichtungen anhand vom Zeichnungen erläutert. Es zeigen:
- Fig. 1:: ein Schema der Vorrichtung mit Darstellung der Beschickung, der Dosierung und ggf. Sperrung sowie der Kontrolle des zu verabreichenden flüssigen Medikaments;
- Fig. 2:: ein Dosierungselement in form eines Wippentasters im Bereich des Schlauchs vom Beschickungsteil zur Infusions- stelle, im Längsschnitt;
- Fig. 3:: das Dosierungselement gemäß Fig. 2 im Querschnitt;
- Fig. 4:: das Dosierungselement gemäß Fig. 2 in Aufsicht;
- Fig. 5:: ein Dosierungselement im Bereich des Schlauchs wie in Fig. 2 bis 4, jedoch mit einem elektronisch gesteuerten Radialmotor zur Betätigung der Klemmung des Zuführungsschlauchs, im Längsschnitt;
- Fig. 6:: das Dosierungselement gemäß Fig. 5 in Aufsicht;
- Fig. 7:: das Dosierungselement im Bereich des Schlauchs wie in Fig. 2 bis 4, jedoch mit einem elektronisch gesteuerten Linearmotor zur Betätigung der Klemmung der Schlauchzuführung, im Längsschnitt;
- Fig. 8:: das Dosierungselment im Bereich des Schlauchs wie in Fig. 2 bis 4, jedoch mit einem elektronisch gesteuerten, nicht erfindungsgemässen Schlauch- ventil zur Veränderung der Durchflußmenge durch den Schlauch, im Längsschnitt;
- Fig. 9:: das Dosierungsefement mit Wippentaster gemäß Fig. 2 bis 4 mit einem uhrwerkgesteuerten Element zur Sperrung des Schlauchdurchflusses, im Querschnitt;
- Fig. 10:: das Dosierungselement mit Sperrungselement gemäß Fig. 9 im Querschnitt;
- Fig. 11:: eine Halterung für die Dosierungselemente gemäß fig. 2 bis 10 für deren Befestigung am menschlichen Arm in Aufsicht;
- Fig. 12:: d i e Halterung für die Dosierungselemente gemäß Fig. 11 im Querschnitt.

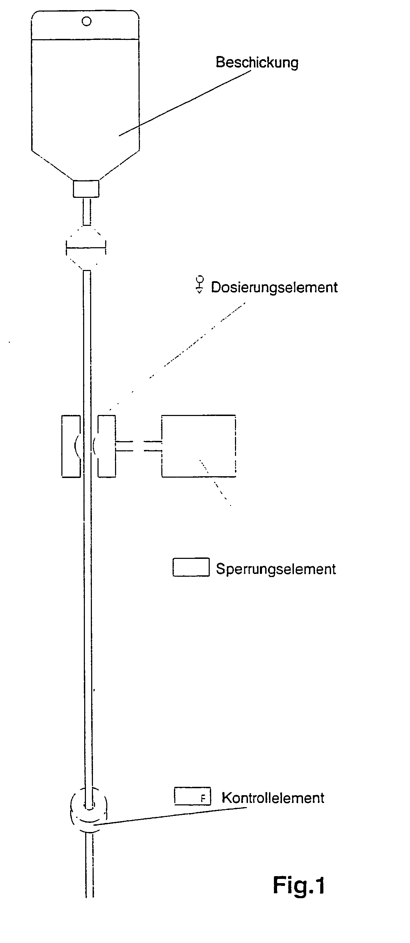

Bei der erfindungsgemäßen Vorrichtung wird, wie in Fig. 1 schematisch dargestellt, das zu verabreichende Medikament, z. B. Schmerzmittel, in flüssiger Form aus einem Behälter zur Bereithaltung über einen Schlauch 4 geleitet, an dessen Ende ein Katheter in das Gefäßsystem des Patienten führt (in der Zeichnung nicht dargestellt). Die Menge des zu verabreichenden Medikaments und der Zeitpunkt der Verabreichung werden im Bereich des Schlauchs 4 dosiert bzw. gesteuert, und zwar sowohl mengenmäßig als auch zeitlich oder alternativ. Dazu wird die Durchflußmenge, d. h. die Menge des bei der Infusion verabreichten Medikaments, durch Klemmung oder Freigabe des Zuführungsschlauchs variiert und durch Messung mit dem Kontrollelement kontrolliert. Die Dosierung der zu verabreichenden Medikamentenmenge sowie ggf. eine Unterbrechung oder völlige Beendigung der Verabreichung durch Sperrung des Zuflusses erfolgen verfahrensgemäß durch unterschiedlich ausgestaltete Klemmelemente.

In den Fig. 2 bis 4 ist ein Dosierungselement in Form eines mechanischen Wippentasters 3 in verschiedenen Ansichten dargestellt. Dieser Wippentaster 3 hat eine Schlauchführung 5, in die der Schlauch 4 an wählbarer Stelle eingepaßt und mittels eines Deckels 6 mit Schrauben 7 im Gehäuse 15 fixiert wird, so daß ein Herausrutschen oder unqualifiziertes Entfernen des Schlauchs 4 aus der Vorrichtung verhindert wird. Der Wippentaster 3 besitzt als Klemmelement eine Wippe 10, die um die Wippenachse 13 mit ihrem einen Längsende gegen den Schlauch 4 drücken und diesen bzw. seine Durchflußöffnung verengen kann bis hin zum vollständigen Verschluß und Sperrung des Durchflusses. Zur Dosierung des Medikamentendurchflusses im Schlauch ist im Gehäuse 15 des Wippentasters 3 eine auf das vorgenannte, auf den Schlauch 4 wirkende, Ende der Wippe 10 drückende Stellschraubenfeder 9 angebracht; durch entsprechende Verstellung der Stellschraube 8 wird die Wippe 10 über die Stellschraubenfeder 9 mehr oder weniger gegen den Schlauch 4 gedrückt und verändert dessen Durchflußquerschnitt bis hin zur vollständigen Sperrung. Zusätzlich zu dieser Dosierungsmöglichkeit hat im dargestellten Ausführungsbeispiel der Wippentaster 3 an seinem entgegengesetzten, nicht auf den Schlauch 4 einwirkenden, Ende der Wippe 10 einen manuell zu betätigenden Auslösertaster 11 mit angesetzter Tasterfeder 12, wobei letztere im Gehäuse 15 befestigt ist und der Auslösertaster 11 durch das Gehäuse und die Tasterfeder 12 auf die Wippe 10 wirkt. Zu einer zusätzlichen, willkürlich zu bewirkenden Medikamentenzufuhr drücken der Patient oder die behandelnde Person den Auslösertaster 11, wodurch die Wippe 10 über ihre Achse 13 den Durchfluß im Schlauch 4 über die Grundeinstellung mittels der Stellschraube 8 weiter bzw. vollständig freigibt. Wird der Auslösertaster 11 wieder freigegeben, wird die Wippe 10 über die Tasterfeder 12 wieder in ihre Grundeinstellung zurückgeführt.

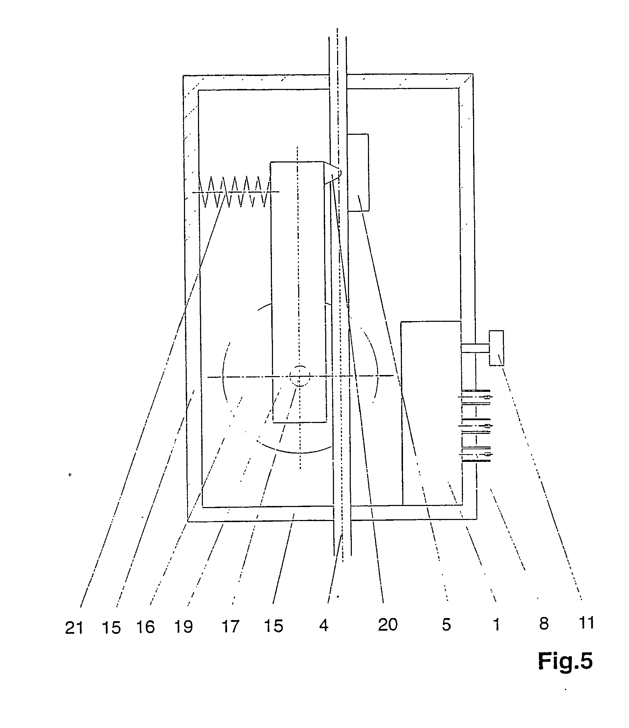

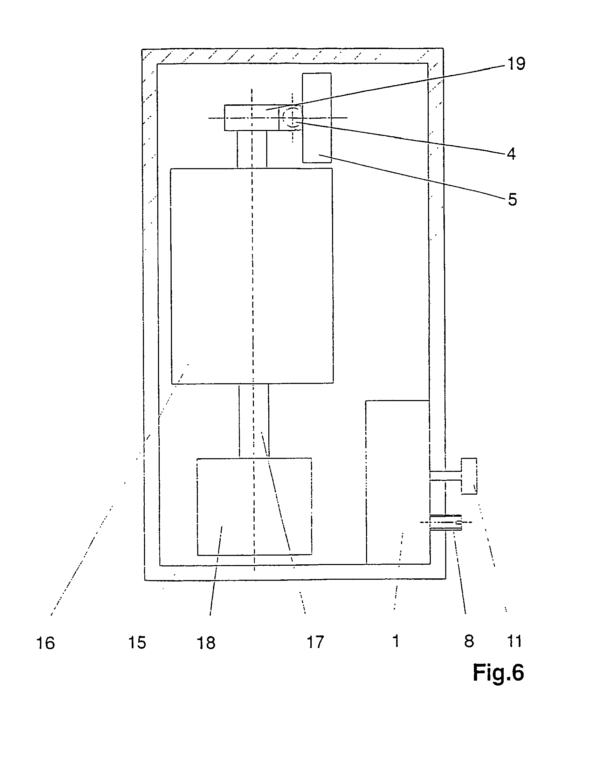

In den Fig. 5 und 6 ist eine andere Ausführungsform des Dosierungselements mit einem elektronisch gesteuerten, elektrisch angetriebenen Radialmotor dargestellt. An dessen verlängerter Drehachse 17 ist ein Klemmhebel 19 fest angebracht, der an seinem entgegengesetzten Ende die nasenförmige Klemmung 20 hat, welche am Schlauch 4 anliegt. Auf der der Klemmung 20 entgegengesetzten Seite des Schlauchs 4 ist dieser in die Schlauchführung 5 eingepaßt. Die Drehachse des Motors 16 verändert mit ihrer Drehung die Stellung des Klemmhebels 19 und damit des Andrucks der Klemmung 20 an den Schlauch 4, der dadurch in seinem Durchflußquerschnitt verändert wird bis zu der völligen Sperrung. Die Regelung des Motors 16 und der Stellung des Klemmhebels 19 erfolgt über eine Elektronik 1, die mit einem Winkelcodierer 18 auf der Drehachse 17 zusammenwirkt, mit dem die Stellung der Motordrehachse 17 bzw. der Klemmung 20 gemessen wird. Zusätzlich ist auch hier ein Auslösertaster 11 vorhanden, der bei der Betätigung durch den Patienten oder Pfleger für eine Boluszugabe die Wirkung der Klemmung, 20 aufhebt. Ferner ist in Fig. 5 für den Fall einer Funktionsstörung des Radialmotors und/oder der Elektronik am Klemmhebel 19 im Bereich der Klemmung 20 eine Rückstellfeder 21 angebracht, die die Klemmung 20 gegen den Schlauch 4 drückt und derart den Durchfluß blockiert. An der Elektronik 1 werden die Basisinfusionsmenge, Gesamthöchstmengen, Bolusmengen des zu verabreichenden Medikaments sowie Totzeiten, d. h. Zeiträume, in denen der Auslösertaster 11 nicht erfolgreich betätigt werden und somit kein Bolus gegeben werden kann, in Abhängigkeit von dem Querschnitt des Schlauchs 4 und des Katheters festgelegt. Im dargestellten Ausführungsbeispiel sind für die Festlegung dieser Werte Stellschrauben 8 am Elektronikteil 1 vorgesehen.

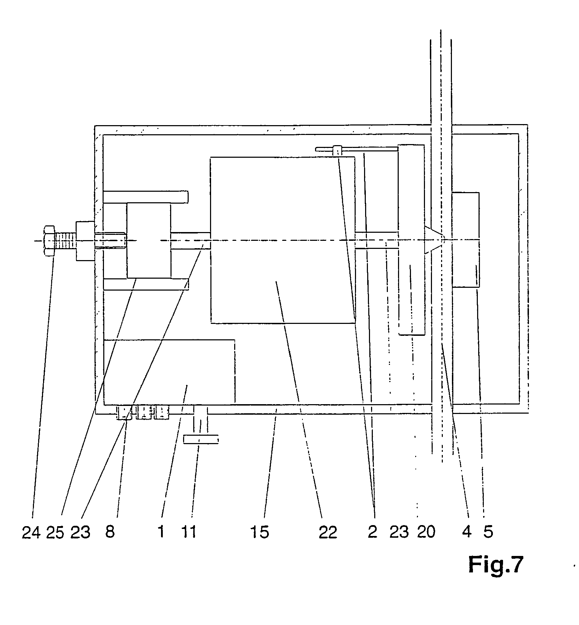

In Fig. 7 ist als weitere Ausführungsform der Vorrichtung ein Dosierungs- und Sperrungselement zur Steuerung der zu verabreichenden Medikamentenmenge dargestellt, bei dem die Klemmung 20 für den Schlauch 4 durch einen Linearmotor oder ein Magnetventil 22 in ihrem Andruck an den Schlauch 4 gesteuert wird, indem die Linearachse 23 mit der Klemmung 20 fest verbunden ist und sich mit dieser und ihrem nasenförmigen Vorsprung in Achsenrichtung entsprechend der Steuerung durch die Elektronik 1 auf den Schlauch 4 zu oder von diesem weg bewegt. Im übrigen weist auch diese Ausführungsform des Dosierungs- und Sperrungselements die Merkmale der Ausführungsform gemäß Fig. 5 und 6 auf, insbesondere auch hinsichtlich der Regelung durch die Elektronik 1, die Stellschrauben 8 und hinsichtlich des Auslösertasters 11. Zusätzlich ist an der Linearachse 23 an ihrem von der Klemmung 20 abgewandten Ende eine Zugstellschraube 24 angeordnet, die skaliert ist und mittels deren die Klemmung 20 vom Schlauch 4 gelöst werden kann, um eine Basisinfusionsmenge einzustellen. Außerdem kann die Stellung der Klemmung 20 mit einem Linearlängenmesser 2 gemessen werden.

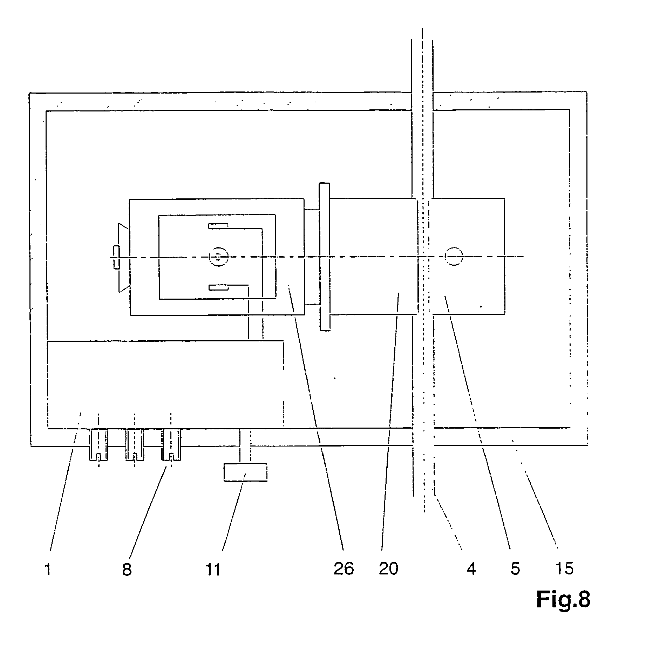

Eine nicht erfindungsgemäße Ausführungsform des Dosierungs- und Sperrungselements unter Verwendung eines Schlauchventils ist in Fig. 8 dargestellt. Dabei ist der Schlauch 4 in einem seinem Durchmesser angepaßten Kanal der Klemmungs 20 des Magnetventils 26 geführt. Das Magnetventil 26 steht wie bei den vorstehend beschriebenen Ausführungsformen mit der Elektronik 1 nebst Stellschrauben 8 und Auslösertaster 11 in Verbindung und wird über diese gesteuert. In Ruhestellung des Schlauchventils 26 wird der Schlauch 4 vollständig geklemmt, so daß ein Medikamentendurchfluß gesperrt ist. Bei Aktivierung des Schlauchventils 26 wird das Lumen des Schlauchs und damit der Durchfluß entsprechend den eingestellten Werten und der Steuerung über die Elektronik 1 freigegeben. Die übrigen Funktionen entsprechen denen der zuvor beschriebenen Ausführungsformen. Indem automatisch in Intervallen kleine Bolusmengen des flüssigen Medikaments verabreicht werden, addieren diese sich zu einer gleichmäßigen Basisinfusionsmenge, die durch Veränderung der Intervallzeiten und der Bolusmengen variiert werden können.

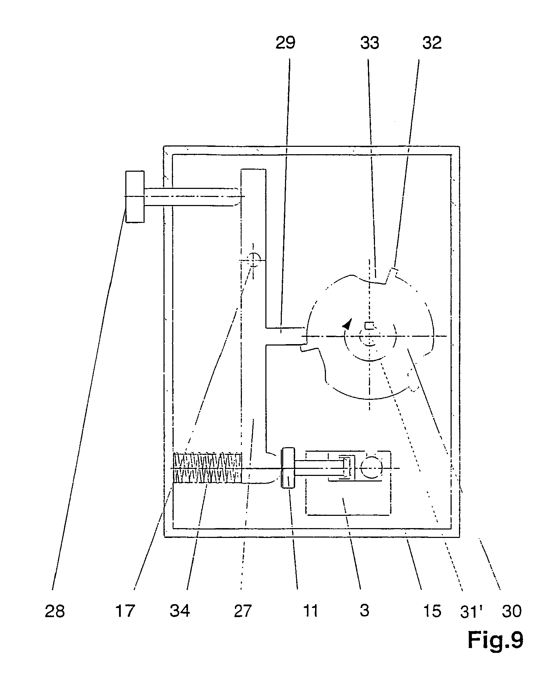

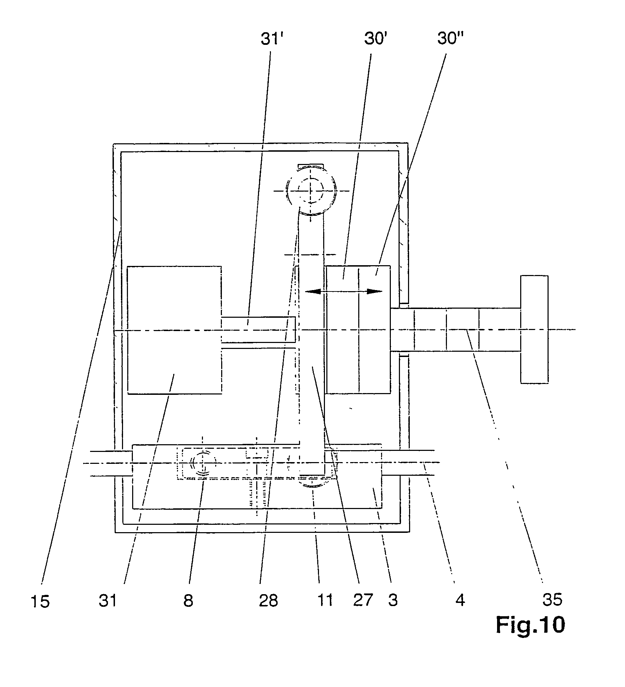

In den Fig. 9 und 10 ist ein zeitgesteuertes Sperrungs- bzw. Unterbrecherelement zur Unterbrechung des Durchflusses des flüssigen Medikaments durch den Schlauch 4 über definierte Zeiträume dargestellt, das mit dem Dosierungselement 3, 10 gemäß Fig. 2 bis 4 zusammenwirkt. Dabei ist ein Rasthebel 27 so in der Vorrichtung angeordnet, daß er mit seinem einen Ende an der Druckfläche des Auslösertasters 11 des Wippentasters 3 anliegt. Im Bereich seines entgegengesetzten Längsendes ist der Rasthebel um eine Achse 17 drehbar gelagert. Zwischen dieser Achse 17 und dem am Auslösertaster 11 anliegenden Ende hat der Rasthebel 27 einen nasenförmigen Vorsprung 29, der an den Außenrand einer Rastscheibe 30 kraftschlüssig angreift. Diese Rastscheibe sitzt auf einer parallel zur Achse 17 und ebenso parallel zum Schlauch 4 verlaufenden Achse 31', die von einem Uhrwerk 31 auf derselben Achse im Uhrzeigersinn bewegt wird. Die Rastscheibe 30 hat eine oder mehrere Ausnehmungen als Rastscheibennuten 33 und im Uhrzeigersinn vor diesen jeweils eine Rastscheibenlippe 32, die über den Scheibenrand übersteht. Der Rasthebel 27 ist im Bereich seiner Anlage am Auslösertaster 11 auf seiner Rückseite mit einer Druckfeder 34 beaufschlagt, die über ihn auf den Auslösertaster Druck ausübt. Der Vorsprung 29 sitzt auf der Rastscheibe 30 auf und verhindert die Eindrückung des Auslösertasters 11 durch die Druckfeder 34 in den Wippentaster 3 und damit eine Öffnung der Klemmung des Schlauchs 4 durch die Stellschraube 8 und die Stellschraubenfeder 9. In dieser Stellung bleibt der Rasthebel 27, bis durch die Drehung der Rastscheibe 30 die Rastscheibenlippe 32 an den Vorsprung 29 anschlägt. Durch Niederdrücken eines Auslösertasters 28 für den Rasthebel 27, der am entgegengesetzten Ende des Rasthebels 27 vor dessen Achse 17 und dem Vorsprung 29 angreift, kann der Rasthebel so angehoben werden, daß der Vorsprung 29 bei weiterer Drehung der Rastscheibe 30 um die Achse 17 nicht mehr von der Rastscheibenlippe 30 blockiert wird. Bei der Weiterbewegung der Rastscheibe 30 durch das Uhrwerk 31 gelangt der Vorsprung 29 in den Bereich der Rastscheibennut 33, so daß nach Freigabe des Auslösertasters 28 der Rasthebel 27 durch die Druckfeder 34 den Auslösertaster 11 eindrücken und die Schlauchzuführung zur Bolusgabe voll öffnen kann. Bei der Weiterdrehung der Rastscheibe 30 gelangt der Vorsprung 29 wieder aus der Nut heraus und wirkt über sie der Rasthebel 27 der Federkraft der Druckfeder 34 entgegen. Dabei wird zugleich die Betätigung des Auslösertasters 28 verhindert und eine Bolusgabe ausgeschlossen. Durch Veränderung der Geschwindigkeit der Drehung der Rastscheibe 30 durch das Uhrwerk 31 oder durch Einsatz anderer Rastscheiben 30', 30" mit unterschiedlicher Anzahl, Form oder geänderter Anordnung der Rastscheibennuten 33 oder mittels einer Rastscheibenverstellung 35 können die Zeitspannen der Unterbrechung der Medikamentenzufuhr verändert werden. Ferner kann durch geänderte Längen der Rastscheibennuten 33 die Zeitdauer der Öffnung des vollen Lumens des Schlauchs 4 für Bolusgaben verändert werden.

Alle Ausführungsformen der Dosierungs- und Sperrungselemente sind in einem Gehäuse 15 angeordnet.

Die Kontrolle der verabreichten Menge des flüssigen Medikaments erfolgt, in den Zeichnungen nicht dargestellt, in an sich bekannter Weise alternativ oder auch kumulativ durch Tropfenzähler, Durchflußmengenzähler oder eine Waage, die im Bereich des Schlauchs 4 vor dem Infusionsteil angeordnet sind. Ebenso können dafür ein Linearpotentiometer am Dosierungselement verwandt werden oder die Sauerstoffsättigung des Bluts des Patienten gemessen werden.

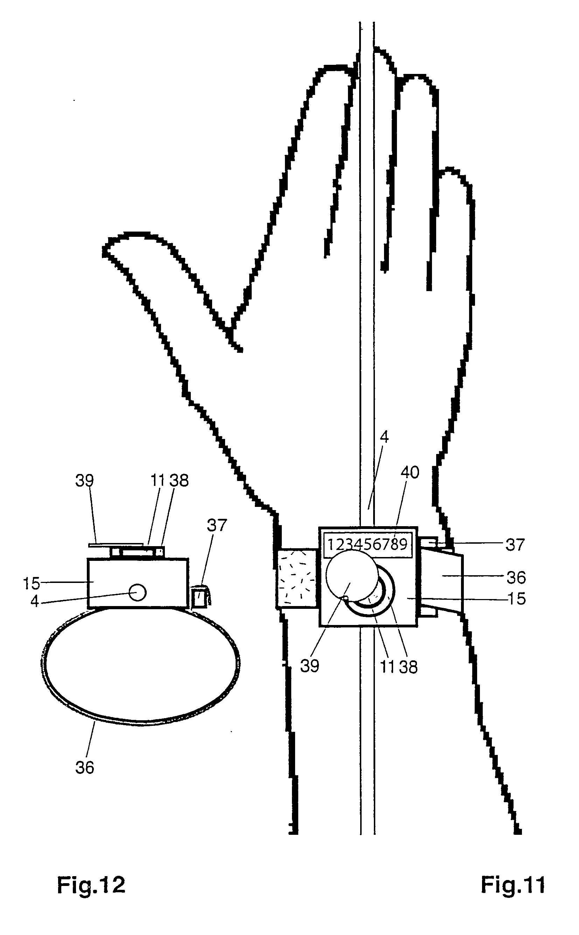

Zur Sicherung des Auslösertasters 11 gegen eine versehentliche Betätigung ist dieser, wie aus Fig. 11 ersichtlich, in einem Schutzring 38 versenkt und mit einem Deckel mit Rückstellfeder 39 abgedeckt.

Zur sicheren Halterung der Dosierungs- und Sperrungselemente bei Anwendung des Verfahrens sind diese Elemente mit einem Halteband 36 nebst Verschluß 37 an ihrem Gehäuse 15 versehen, mit dem die Elemente am Arm des Patienten, am Infusionsständer, am Bettgalgen oder an anderer geeigneter Stelle so angebracht werden können, daß der Auslösertaster 11 für den Patienten sicher erreichbar ist (Fig. 11 und 12).

In zweckmäßiger Ausführungsform ist, wie ebenfalls aus Fig. 11 ersichtlich, im Gehäuse 15 ein Display 40 angeordnet, auf dem z. B. die Anzahl der erfolgreichen oder erfolglosen Betätigungen des Auslösertasters 11 oder sonstige Angaben zur Patientengeschichte dargestellt werden.

Die erfindungsgemässen Vorrichtungen ermöglichen eine Steuerung von außen her in Bezug auf die Dosierung des Medikaments und der Applikationsgeschwindigkeit der Medikamentengabe. Es werden eine definierte Basisinfusionsgeschwindigkeit ebenso möglich wie definierte Bolusgaben. Zudem wird die Vorgabe von Zeiträumen für die Sperrung einer Medikamentenabgabe bzw. Bolusgabe und die Vorgabe von Mengenbeschränkungen der Verabreichung des Medikaments, für bestimmte Zeiträume möglich. Dabei können die Vorrichtungen unter Einsatz eines traditionellen Infusions. oder Transfusionssystems zur Verabreichung der Medikamente eingesetzt werden, wobei das Medikament, z. B. Schmerzmittel, der Infusionslösung zugegeben wird. Es werden keine aufwendigen Transportsysteme benötigt, z. B. in Form einer Präzisionspumpe, keine gesonderten Vorratsbehälter mit Verbindungsleitungen und keine Rückschlagventile. Es kann ohne zusätzliches spezielles Einmalmaterial ausgeführt werden. Gleichwohl wird die Variabilität der Menge und der Zeitpunkte sowie der Zeiträume für die Verabreichung des Medikaments vergrößert. Auch ist es möglich, die Technik der Vorrichtungen so einfach zu gestalten, daß im Vergleich zu bekannten patientenkontrollierten Dosiersystemen nur ein Bruchteil der Herstellungskosten anfällt und daß auch ein stark vereinfachtes Bedienkonzept angewandt werden kann, das vor allem nicht speziell geschultem medizinischem Personal ermöglicht, die Beschickung der Vorrichtungen und die Grundeinstellung vorzunehmen. Die hier beschriebenen Vorrichtungen verzichten auf Präzisionsantriebe und nehmen gegenüber bekannten Vorrichtungen bewußt die Möglichkeit einer weniger präzisen Dosierung in Kauf. Es ist daher insbesondere in der Akutschmerztherapie, z. B. bei postoperativen Schmerzzuständen, einsetzbar, wo die Schmerzstärke individuell variabel und nicht vorhersagbar ist und sich im Verlauf stark ändert, was eine präzise patientengerechte Grundeinstellung ohnehin unmöglich macht. Bei Verwendung der vorgeschlagenen Ausführungsformen der Dosierungselemente mit elektronischer Steuerung wird je nach Anwendung eine Einstellung mit vielen Sperr- und Kontrollwerten ermöglicht oder eine einfachere mit weniger Werten und damit den besonderen Bedingungen des einzelnen Patienten anpaßbare Verabreichung des Medikaments möglich. Die Einstellungen, der aktuelle Medikamentenverbrauch und die- erfolgreichen oder infolge der Sperrung erfolglosen - Betätigungen des Auslösertasters sind auf einem Display sichtbar.

### BEZUGSZEICHENLISTE

1. Elektronik
2. Linearlängenmesser
3. Wippentaster
4. Schlauch
5. Schlauchführung
6. Deckel
7. Verschraubung
8. Skalierte Stellschraube (Einstellelemente)w
9. Stellschraubenfeder
10. Wippe
11. Auslösertaster
12. Auslösertasterfeder
13. Wippenachse
14. Gurtschlitz
15. Gehäuse
16. Radialmotor
17. Drehachse
18. Winkelkodierer
19. Klemmhebel
20. Klemmung
21. Rückstellfeder
22. Linearmotor/Magnetventil
23. Linearachse
24. Skalierte Zugstellschraube
25. Befestigung
26. Schlauchventil
27. Rasthebel
28. Rasthebelauslösetaster
29. Rasthebelnase
30. Rastscheibe (30', 30")
31. Uhrwerk (31'. Achse Uhrwerk-Rastscheibe)
32. Rastscheibenlippe
33. Rastscheibennut
34. Druckfeder
35. Rastscheibenverstellung
36. Halteband
37. Verschluß
38. Schutzring
39. Deckel mit Rückstellfeder
40. Display

## Patentansprüche

1. Vorrichtung zum Selbstdosieren und zur Steuerung, d. h. Begrenzung und Sperrung, der Dosierung der Verabreichung eines flüssigen Medikaments umfassend einen Behälter, einen Schlauch und einen Katheter, wobei das zu verabreichende Medikament, z. B. Schmerzmittel, sich in dem Behälter befindet und unter Einsatz eines herkömmlichen Infusions- oder Transfusionssystems, vorzugsweise schwerkraftgetrieben ohne Transportsystem z. B. in Form einer zusätzlichen Pumpe, über den Schlauch und den Katheter an seinem Ende in das Gefäßsystem des Patienten eingeführt wird,
wobei die Vorrichtung ein willkürlich von Hand umschaltbares Dosierungselement mit einem Auslöserelement (11) und einem Klemmelement (10) umfaßt,
wobei das Dosierungselement außen am Schlauch (4) angebracht ist und
wobei das Auslöserelement (11) manuell willkürlich betätigbar ist und in Wirkverbindung mit einem einstellbaren Klemmelement (10) steht, wodurch die Durchflußöffnung des Schlauchs (4) und damit die Durchflußmenge des zu verabreichenden Medikaments zeitlich befristet verändert werden, wobei die Durchflußöffnung des Schlauchs (4) sowohl im Grundzustand, z. B. geschlossen oder halb geöffnet, als auch im Boluszustand, z. B. ganz geöffnet, willkürlich durch eine Stellschraube (8), definiert werden kann,
**dadurch gekennzeichnet,**
- **daß** das Auslöseelement ein Auslösertaster ist,
- und **daß** der Auslösertaster (11) eine angesetzte Tasterfeder (12) hat, die im Gehäuse (15) des Dosierungselements befestigt ist,
- und **daß** das Dosierungselement einen mechanischen Wippentaster (3) aufweist, der derart an den Schlauch (4) an wählbarer Stelle angesetzt ist, daß der Wippentaster (3) eine Schlauchführung (5) hat, in die der Schlauch (4) eingepaßt und mittels eines Deckels (6) mit Schrauben (7) im Gehäuse (15) des Dosierungselements fixiert wird,
- und **daß** der Wippentaster (3) als Klemmelement eine Wippe (10) hat, wobei im Betrieb die Wippe (10) um die Wippenachse (13) mit ihrem einen Längsende gegen den Schlauch (4) drücken und diesen in seiner Durchflußöffnung verengen kann bis zum vollständigen Verschluß und Sperrung des Durchflusses, indem eine Stellschraubenfeder (9) auf das am Schlauch anliegende Ende der wippe (10) wirkt, und daß ferner die Wippe durch verstellung der Stellschraube (8) an der Stellschraubenfeder (9) in ihrem Andruck an den Schlauch (4) geregelt wird und dessen Durchflußquerschnitt bis zur vollständigen Sperrung verändert, und wobei der Auslösertaster (11) durch das Gehäuse und die Tasterfeder (12) auf die Wippe (10) wirkt und durch Bestätigung des Auslösertasters (11) der Durchfluß im Schlauch (4) vergrößert oder vollständig freigegeben werden kann.

2. Vorrichtung nach Patentanspruch 1, **dadurch gekennzeichnet, daß** des Auslösertaster (11) mit angesetzter Tastfeder (12) an dem nicht auf den Schlauch (4) einwirkenden Ende der wippe (10) des wippentasters (3) angeordnet ist.

3. Vorrichtung zum Selbstdosieren und zur Steuerung, d. h. Begrenzung und Sperrung, der Dosierung der Verabreichung eines flüssigen Medikaments umfassend einen Behälter, einen Schlauch und einen Katheter, wobei das zu verabreichende Medikament, z. B. Schmerzmittel, sich in dem Behälter befindet und unter Einsatz eines herkömmlichen Infusions- oder Transfusionssystems, vorzugsweise schwerkraftgetrieben ohne Transportsystem z. B. in Form einer zusätzlichen Pumpe, über den Schlauch und den Katheter an seinem Ende in das Gefäßsystem des Patienten eingeführt wird,
wobei die Vorrichtung ein willkürlich von Hand umschaltbares Dosierungselement mit einem Auslöserelement (11) und einem Klemmelement (19) umfaßt, wobei das Dosierungselement außen am Schlauch (4) angebracht ist und
wobei das Auslöserelement (11) manuell willkürlich betätigbar ist und in Wirkverbindung mit einem einstellbaren Klemmelement (19) steht, wodurch die Durchflußöffnung des Schlauchs (4) und damit die Durchflußmenge des zu verabreichenden Medikaments zeitlich befristet verändert werden, wobei die Durchflußöffnung des Schlauchs (4) sowohl im Grundzustand, z. B. geschlossen oder halb geöffnet, als auch im Boluszustand, z. B. ganz geöffnet, willkürlich definiert werden kann,
**dadurch gekennzeichnet,**
- **daß** das Auslöseelement ein Auslösertaster ist,
- und **daß** eine Schlauchführung (5) vorhanden ist, in die der Schlauch (4) eingepaßt ist,
- und **daß** auf den Schlauch eine nasenförmige klemmung (20) von außen einwirkt,
- wobei das Klemmelement ein klemmhebel (19) ist, und die klemmung (20) am Ende des klemmhebels angebracht ist und in seiner Position zum Schlauch verstellt werden kann durch das Dosierungselement,
- und **daß** das Dosierungselement ferner einen Radialmotor (16) aufweist, an dessen verlängerter Drehachse (17) der klemmhebel (19) fest angebracht ist,
- und **daß** dieser Radialmotor (16) elektrisch angetrieben und elektronish gesteuert wird durch eine zugehörige steuer-elektronik (1), die mit einem Winkelcodierer (18) auf einer Drehachse (17) zusammenwirkt, mit dem die Stellung de Motordrehachse (17) bzw. der klemmung (20) gemessen wird,
- und **daß** an dieser Steuerelektronik (1) außerdem der Auslösetaster (11) angebracht ist, der bei seiner Betätigung die Wirkung des klemmelements (20) aufheben kann.

4. Vorrichtung nach Patentanspruch 3, **dadurch gekennzeichnet, daß** an des Elektronik (1) Stellschrauben (8) zur Dosierung der medikamentenmenge und Einstellung von mengenbegrenzungen und/oder Zeitbegrenzungen für die medikamentengabe angeordnet sind.

5. Vorrichtung nach Patentanspruch 3, **dadurch gekennzeichnet, daß** am klemmhebel (19) im Bereich der klemmung (20) eine Rückstellfeder (21) angebracht ist, die diese im Falle einer Funktionsstörung des Motors (16) oder der Elektronik (1) gegen den Schlauch drückt und den Duschfluß sperrt.

6. Vorrichtung zum Selbstdosieren und zur Steuerung, d. h. Begrenzung und Sperrung, der Dosierung der Verabreichung eines flüssigen Medikaments umfassend einen Behälter, einen Schlauch und einen Katheter, wobei das zu verabreichende Medikament, z. B. Schmerzmittel, sich in dem Behälter befindet und unter Einsatz eines herkömmlichen Infusions- oder Transfusionssystems, vorzugsweise schwerkraftgetrieben ohne Transportsystem z. B. in Form einer zusätzlichen Pumpe, über den Schlauch und den Katheter an seinem Ende in das Gefäßsystem des Patienten eingeführt wird,
wobei die Vorrichtung ein willkürlich von Hand umschaltbares Dosierungselement
mit einem Auslöserelement (11) und einem Klemmelement umfaßt, wobei das Dosierungselement außen am Schlauch (4) angebracht ist und
wobei das Auslöserelement (11) manuell willkürlich betätigbar ist und in Wirkverbindung mit einem einstellbaren Klemmelement steht, wodurch die Durchflußöffnung (der Querschnitt) des Schlauchs (4) und damit die Durchflußmenge des zu verabreichenden Medikaments zeitlich befristet verändert werden, wobei die Durchflußöffnung des Schlauchs (4) sowohl im Grundzustand, z. B. geschlossen oder halb geöffnet, als auch im Boluszustand, z. B. ganz geöffnet, willkürlich definiert werden kann,
**dadurch gekennzeichnet,**
- **daß** das Auslöseelement ein Auslösertaster ist,
- und dass eine Schlauchführung (5) vorhanden ist, in die der Schlauch (4) eingepasst ist,
- und dass das Klemmelement eine Linearachse (23) ist
- wobei eine nasenförmige Klemmung (20) am Ende der Linearachse (23) angebracht ist und in ihrer Position zum Schlauch verstellt werden kann durch das Dosierungselement und dass auf den Schlauch die klemmung von außen einwirken kann,
- und dass das die Dosierungselement ferner einen Linearmotor oder ein Magnetventil (22) aufweist, womit die Linearachse (23) zum Schlauch bewegt werden kann,
- und dass die Regelung des Linearmotors bzw. Magnetventils (22) und des Andrucks an den Schlauch (4) über die Steuerelektro-nik (1) erfolgt,
- und dass an dieser Steuerelektronik (1) außerdem der Auslösetaster (11) augebracht ist, der bei seiner Betätigung die Wirkung des Klemmhebels (20) aufheben kann.

7. Vorrichtung Patentanspruch 6, **dadurch gekennzeichnet, daß** an der Linearachse (23) an ihrem von dem Klemmelement (20) abgewandten Ende eine skalierte Zugstellschraube (24) angeordnet ist, mittels deren das Klemmelement (20) vom Schlauch (4) gelöst werden kann zur Einstellung einer Basisinfusionsmenge.

8. Vorrichtung nach Patentanspruch 1, **dadurch gekennzeichnet, dass** das Dosierungsgerät nach Anspruch 1 kombiniert ist mit einem Steuerung- bzw. Sperrungs- bzw. Unterbrecherelement (17, 27 bis 34) zur Unterbrechung des Durchflusses eines flüssigen Medikaments durch den Schlauch (4) über 2
einen definierten Zeitraum, wobei das Steuerung- bzw. Sperrungs- bzw. Unterbrecherelement im Betrieb mit dem Dosierungselement zur Unterbrechung des Durchflusses des flüssigen Medikaments durch den Schlauch (4) über definierte'Zeiträume zusammenwirken kann,
, wobei ein Rasthebel (27) mit seinem einen tnde an der Druckfläche des Auslösertasters (11) des Wippentaster (3) anliegt und im Bereich seines entgegengesetzten Längsendes um eine Achse (17) drehbar gelagert ist, daß ferner zwischen dieser Achse (17) und dem am Auslösertaster (11) anliegenden Ende der Rasthebel (27) einen nasenförmigen Vorsprung (29) hat, der seinerseits an den Außenrand einer Rastscheibe (30) kraftschlüssig angreift, daß ferner die Rastscheibe (30) auf einer parallel zur Achse (17) und zum Schlauch (4) verlaufenden Achse (31') sitzt, die von einem Uhrwerk (31) auf derselben Achse bewegt wird, wobei die Rastscheibe (30) eine oder mehrere Rastscheibennuten. (33) mit davor am Außenrand der Rastscheibe (30) angeordneten Rastscheibenlippen (32) hat und der Rasthebel (27) auf seiner dem Auslösertaster (11) gegenüberliegenden Seite mit einer Druckfeder (34) beaufschlagt ist, die den Auslösertaster (11) über den Rasthebel (27) auslöst und **dadurch** den Durchfluß durch den Schlauch (4) voll öffnet, sobald und solange der Vorsprung (29) des Rasthebels (27) während der Drehung der Rastscheibe (30) in einer der Rastscheibennuten (33) läuft, und daß schließlich ein weiterer Auslösertaster (28), der im Gehäuse (15) der Vorrichtung angeordnet ist und am Rasthebel (27) an dessen vor der Achse (17) und an dem dem Vorsprung (29) entgegengesetzten Ende angreift, durch Niederdrücken den Vorsprung (29) anhebt und **dadurch** die Sperre durch die Rastscheibenlippe (32) aufhebt.

9. Vorrichtung nach Patentanspruch 8, **dadurch gekennzeichnet, daß** der Rasthebel (27) über den Vorsprung (29) und den äußeren Rand der Rastscheibe (30) gegen die Druckfeder (34) vom Auslösertaster (11) wegbewegt wird und dieser in seine Ausgangsstellung zurückkehrt, nachdem der Vorsprung (29) eine Rastscheibennut (33) verlassen hat, und daß der Rasthebel (27) und der Auslösertaster (11) in dieser Position solange verbleiben und insbesondere letzterer nicht ausgelöst werden kann, bis die Rastscheibe (30) bei ihrer weiteren Drehung mit einer Rastscheibenlippe (32) an den Vorsprung (29) des Rasthebels (27) anstößt und der Auslösertaster (28) gedrückt wird.

10. Vorrichtung nach Patentanspruch 8, **dadurch gekennzeichnet, daß** durch Veränderung der Drehgeschwindigkeit der Rastscheibe (30) durch das Uhrwerk (31) oder mittels Einsatzes von Rastscheiben (30', 30") mit unterschiedlicher Anzahl, Form und Anordnung der Rastscheibennuten (33) oder mittels einer Rastscheibenverstellung (35) die Zeitspannen der Sperrung der Betätigung des Auslösertasters (11) und der Aufhebung der Drosselung des Durchflusses durch den Schlauch (4) verändert werden.

11. Vorrichtung nach einem oder mehreren der Patentansprüche 1 bis 9, **dadurch gekennzeichnet, daß** die Kontrolle der verabreichten Menge des flüssigen Medikaments durch Tropfenzähler, Durchflußmengenzähler oder eine Waage erfolgt, die sämtlich im Bereich des Infusionssystems (Vorratsbehälter und Schlauch) angeordnet sind, und/oder über die Messung der Sauerstoffsättigung des Bluts des Patienten erfolgt.

12. Vorrichtung nach einem oder mehreren der Patentansprüche 1, 3 und 6, **dadurch gekennzeichnet, daß** der Auslösertaster (11) in einem Schutzring (38) eingelassen und mit einem Deckel (39) mit Rückstellfeder abgedeckt ist.

13. Vorrichtung nach einem oder mehreren der Patentansprüche 1 bis 13, **dadurch gekennzeichnet; daß** das Dosierungselement mit einem Halteband (36) nebst Verschluß (37) an ihrem Gehäuse (15) versehen ist, mit dem das Dosierüngselement am Arm des Patienten, an einem Infusionsständer oder an anderer geeigneter Stelle angebracht werden können.

14. Vorrichtung nach einem oder mehreren der Patentansprüche 1 bis 14, **dadurch gekennzeichnet, daß** im Gehäuse (15) des Dosierungselements ein Display (40) angeordnet ist, der die Anzahl von Betätigungen des Auslösertasters (11) oder sonstige Angaben zum Patienten und zur Medikamentengabe darstellt.

## Claims

1. Apparatus to self-dose and to control, i.e. limit and block, the dispensing of a liquid medicament, consisting of a container, a hose and a catheter, whereby the medicament to be dispensed, e.g. a painkiller, is held in the container and is fed into the patient's vascular system using a normal infusion or transfusion system - preferably driven by gravity without transport system, e.g. in the form of an additional pump - via the hose and the catheter at its end,
whereby the apparatus consists of a dosing element able to be switched manually at will with a trigger element (11) and a pinching element (10),
whereby the dosing element is fitted on the outside to the hose (4) and
whereby the trigger element (11) is able to be actuated manually at will and interacts with an adjustable pinching element (10), which enables the throughflow opening of the hose (4) and thus the throughflow volume of the medicament to be dispensed to be temporarily altered, whereby the throughflow opening of the hose (4) can be defined at will, both in the basis state, e.g. closed or half opened, and in the bolus state, e.g. fully opened, by means of a set screw (8),
**characterised in that**
- the trigger element is a trigger switch,
- and that the trigger switch (11) is attached to a tracer spring (12) which is fastened in the housing (15) of the dosing element,
- and that the dosing element has a mechanical rocker switch (3) which is attached to the hose (4) in a selectable place in such a way that the rocker switch (3) has a hose guide (5) into which the hose (4) is inserted and fixed in the housing (15) of the dosing element by means of a cover (6) with screws (7),
- and that the rocker switch (3) as pinching element has a rocker (10), whereby the rocker (10) around the rocker axis (13) presses with one of its longitudinal ends against the hose (4) during operation and can narrow the throughflow opening of the hose until the throughflow is completely closed and blocked, **in that** a set screw spring (9) works on the end of the rocker (10) resting on the hose, and that the pressure applied by the rocker on the hose (4) is furthermore regulated by adjusting the set screw (8) on the set screw spring (9), and alters the throughflow cross-section until complete closure, and whereby the trigger switch (11) works on the rocker (10) through the housing and the tracer spring (12) and the throughflow in the hose (4) can be increased or fully released by actuating the trigger switch (11)

2. Apparatus in accordance with patent claim 1, **characterised in that** the trigger switch (11) with attached tracer spring (12) is arranged on the end of the rocker (10) of the rocker switch (3) not acting upon the hose (4).

3. Apparatus to self-dose and to control, i.e. limit and block, the dispensing of a liquid medicament, consisting of a container, a hose and a catheter, whereby the
medicament to be dispensed, e.g. a painkiller, is held in the container and is fed into the patient's vascular system using a normal infusion or transfusion system - preferably driven by gravity without transport system, e.g. in the form of an additional pump - via the hose and the catheter,
whereby the apparatus consists of a dosing element able to be switched manually at will with a trigger element (11) and a pinching element (10),
whereby the dosing element is fitted on the outside to the hose (4) and
whereby the trigger element (11) is able to be actuated manually at will and interacts with an adjustable pinching element (19), which enables the throughflow opening of the hose (4) and thus the throughflow volume of the medicament to be dispensed to be temporarily altered, whereby the throughflow opening of the hose (4) can be defined at will, both in the basis state, e.g. closed or half opened, and in the bolus state, e.g. fully opened,
**characterised in that**
- the trigger element is a trigger switch,
- and that there is a hose guide (5) into which the hose (4) is inserted,
- and that a nose-shaped pinch (20) acts upon on the hose from the outside,
- whereby the pinching element is a pinching lever (19), and the pinch 20 is fitted to the end of the pinching lever and which can be adjusted in its position to the hose by the dosing element,
- and that the dosing element furthermore has a radial motor (16), on whose extended rotary axis (17) the pinching lever (19) is permanently attached,
- and that this radial motor (16) is driven by electricity and controlled electronically by an associated electronic control unit (1), which interacts with an angle coder (18) on an rotary axis (17), with which the position of the motor's rotary axis (17) resp. of the pinch (20) is measured,
- and that the trigger element (11) is also fitted to this electronic control unit (1), that when actuated can nullify the effect of the pinching element (20).

4. Apparatus in accordance with patent claim 3, **characterised in that** set screws (8) are arranged on the electronic control unit (1) for dosing the volume of the medicament and adjusting volume limitations and/or time limitations for dispensing the medicament,

5. Apparatus in accordance with patent claim 3, **characterised in that** a return spring (21) is arranged on the pinching lever (19) around the pinch (20), which presses this against the hose (4) and blocks the throughflow in case of a functional disturbance in the motor (16) or in the electronic control unit (1).

6. Apparatus to self-dose and to control, i.e. limit and block, the dispensing of a liquid medicament, consisting of a container, a hose and a catheter, whereby the medicament to be dispensed, e.g. a painkiller, is held in the container and is fed into the patient's vascular system using a normal infusion or transfusion system - preferably driven by gravity without transport system, e.g. in the form of an additional pump - via the hose and the catheter,
whereby the apparatus consists of a dosing element able to be switched manually at will with a trigger element (11) and a pinching element,
whereby the dosing element is fitted on the outside to the hose (4) and
whereby the trigger element (11) is able to be actuated manually at will and interacts with an adjustable pinching element, which enables the throughflow opening (cross-section) of the hose (4) and thus the throughflow volume of the medicament to be dispensed to be temporarily altered, whereby the throughflow opening in the hose (4) can be defined at will, both in the basis state, e.g. closed or half opened, and in the bolus state, e.g. fully opened,
**characterised in that**
- the trigger element is a trigger switch,
- and that there is a hose guide (5) into which the hose is inserted,
- and that the pinching element is a linear axis (23)
- whereby a nose-shaped pinch (20) is fitted at the end of the linear axis (23) and can be adjusted in its position to the hose by the dosing element and that the pinch can act upon the hose from the outside,
- and that the dosing element furthermore has a linear motor or a solenoid valve (22), with which the linear axis (23) can be moved to the hose
- and that the electronic control unit (1) regulates the linear motor or solenoid valve (22) and the pressure on the hose (4),
- and that the trigger element (11) is furthermore fitted on this electronic control unit (1), that when actuated can nullify the effect of the pinching element (20).

7. Apparatus in accordance with patent claim 6, **characterised in that** a scaled tensioning set screw (24) is arranged on the linear axis (23) on its end turned away from the pinching element (20), which enables the pinching element (20) to be undone from the hose (4) in order to adjust a basis infusion volume.

8. Apparatus in accordance with patent claim 1, **characterised in that** the dosing device as per claim 1 is combined with a control / blockage / interrupter element (17, 27 to 34) to interrupt the throughflow of a liquid medicament through the hose (4) over a defined period of time, whereby the control / blockage / interrupter element can interact over defined periods of time during operation with the dosing element to interrupt the throughflow of the liquid medicament through the hose (4),
whereby one end of a latching lever (27) rests on pressure surface of the trigger switch (11) of the rocker.switch (3) and is rotary mounted around its opposite longitudinal end around an axis (17), that it furthermore has a nose-shaped projection (29) between this axis (17)
and the end of the latching lever (27) resting on the trigger switch (11), which in turn positively locks into the outer edge of a latching disk (30), that furthermore the latching disk (30) sits on an axis (31') running parallel to the axis (17) and to the hose (4) which is moved by clock mechanism (31) on the same axis, whereby the latching disk (30) has one or more latching disk grooves (33) with latching disk lips (32) in front of them arranged on the outer edge of the latching disk (30) and the latching lever (27) has on its side opposite the trigger switch (11) a pressure spring (34) which actuates the trigger switch (11) via the latching lever (27) and thereby fully opens the throughflow through the hose (4), as soon and as long as the projection (29) of the latching lever (27) runs into one of the latching disk grooves (33) whilst the latching disk (30) turns, and that finally a further trigger switch (28) - which is arranged in the apparatus housing (15) and which engages the latching lever (27) in front of its axis (17) and on the end opposite the projection (29) - lifts the projection (29) by pressing it down and thereby releases the blockage caused by the latching disk lip (32).

9. Apparatus in accordance with patent claim 8, **characterised in that** the latching lever (27) is moved away from the trigger switch (11) by the projection (29) and the outer edge of the latching disk (30) against the pressure spring (34) and the trigger switch returns to its home position, after the projection (29) has left a latching disk groove (33), and that the latching lever (27) and the trigger switch (11) remain in this position as long as and in particular the latter cannot trigger until the latching disk (30) whilst turning further catches with a latching disk lip (32) on the projection (29) of the latching lever (27) and the trigger switch (28) is pressed down.

10. Apparatus in accordance with patent claim 8, **characterised in that** the closure intervals in the actuation of the trigger switch (11) and the nullification of the throttling of the throughflow through the hose (4) can be altered by changing the rotary speed of the latching disk (30) by the clockwork mechanism (31) or by deploying latching disks (30', 30") with differing number, form and arrangement of latching disk grooves (33) or by a latching disk adjustment (35).

11. Apparatus in accordance with one or more of the patent claims 1 to 9, **characterised in that** the volume of the liquid medicament dispensed is controlled by a drop counter, throughflow volume counter or scales, all arranged around the infusion system (supply container and hose), and/or is controlled by measuring the oxygen saturation in the patient's blood.

12. Apparatus in accordance with one or more of the patent claims 1, 3 and 6, **characterised in that** the trigger switch (11) is inserted in a protective ring (38) and is covered by a cover (39) with return spring.

13. Apparatus in accordance with one or more of the patent claims 1 to 13, **characterised in that** the dosing element is provided with a retainer band (36) plus lock (37) on its housing (15), with which the dosing element can be attached to the patient's arm, to an infusion stand or to some other suitable place.

14. Apparatus in accordance with one or more of the patent claims 1 to 14, **characterised in that** a display is installed in the housing (15) of the dosing element, which displays the number of times that the trigger switch (11) has been actuated or other details on the patient and the dispensing of the medicament.

## Revendications

1. Dispositif d'autodosage et de réglage, c-à-d de limitation et d'arrêt, du dosage de l'administration d'un médicament liquide, comprenant un contenant, un tuyau et un cathéter, pour lequel le médicament à être administer, par ex. un analgésique, se trouve dans le contenant et est introduit dans le système vasculaire du patient par la mise en oeuvre d'un système de perfusion ou de transfusion conventionnel, de préférence fonctionnant par gravité, sans système de transport, par ex. en forme d'une pompe supplémentaire, au moyen du tuyau et du cathéter situé à son extrémité,
le dispositif comportant un élément de dosage pouvant être réglé à volonté manuellement et doté d'un élément de déclenchement (11) et d'un élément de serrage (10),
l'élément de dosage étant disposé sur la partie extérieure du tuyau (4)
et l'élément de déclenchement (11) pouvant être actionné manuellement à volonté et étant en liaison mécanique avec un élément de serrage (10) réglable, ce qui a pour effet que l'orifice d'écoulement du tuyau (4) et par conséquent le flux du médicament à être administré sont modifiés temporairement; l'orifice d'écoulement du tuyau (4), aussi bien en position initiale, par ex. fermé ou à demi ouvert, qu'à l'état de bolus, par ex. complètement ouvert, pouvant être détérmine à volonté par une vis de réglage (8),
**caractérisé en ce que**
- l'élément de déclenchement est un bouton de déclenchement
- et que le bouton de déclenchement (11) comporte un ressort (12) fixé dans le boîtier (15) de l'élément de dosage,
- et que l'élément de dosage comporte un bouton mécanique à tapecul (3) disposé sur le tuyau (4) à un endroit pouvant être choisi, de sorte que le bouton à tapecul (3) possède un embout d'introduction (5) dans lequel le tuyau (4) est inséré et fixé dans le boîtier (15) de l'élément de dosage, à l'aide d'un couvercle (6) avec des vis (7),
- et que le bouton (3) à tapecul, en tant qu'élément de serrage, présente un tapecul (10); pendant le fonctionnement, le tapecul (10) exerce une pression sur le tuyau (4) avec son extrémité longitudinale autour de l'axe (13) du tapecul et peut rétrécir l'orifice d'écoulement dudit tuyau jusqu'à fermeture compléte et blocage du flux, **en ce qu'**un ressort de vis de réglage (9) agit sur l'extrémité du tapecul (10) disposé sur le tuyau et qu'en outre la pression du tapecul sur le tuyau (4) est réglée par ajustage de la vis de réglage (8) sur le ressort (9) de la vis de réglage, ce qui modifie son gabarit d'écoulement jusqu'à blocage complet, le bouton de déclenchement (11) agissant sur le tapecul (10) par l'intermédiaire du boîtier et du ressort du bouton (12) et par actionnement du bouton (11) le flux dans le tuyau (4) est augmenté ou peut être complètement libéré.

2. Dispositif selon la revendication 1, **caractérisé en ce que** le bouton de déclenchement (11) avec son ressort (12) fixé est disposé à l'extrémité du tapecul (10) du bouton à tapecul (3) n'agissant pas sur le tuyau (4).

3. Dispositif d'autodosage et de réglage, c-à-d de limitation et d'arrêt, du dosage de l'administration d'un médicament liquide, comprenant un contenant, un tuyau et un cathéter, pour lequel le médicament à être administer, par ex. un analgésique, se trouve dans le contenant et est introduit dans le système vasculaire du patient par la mise en oeuvre d'un système de perfusion ou de transfusion conventionnel, de préférence fonctionnant par gravité, sans système de transport, par ex. en forme d'une pompe supplémentaire, au moyen du tuyau et du cathéter situé à son extrémité,
le dispositif comportant un élément de dosage pouvant être réglé à volonté manuellement et doté d'un élément de déclenchement (11) et d'un élément de serrage (19),
l'élément de dosage étant disposé sur la partie extérieure du tuyau (4)
et l'élément de déclenchement (11) pouvant être actionné manuellement à volonté et étant en liaison mécanique avec un élément de serrage (10) réglable, ce qui a pour effet que l'orifice d'écoulement du tuyau (4) et par conséquent le flux du médicament à être administré sont modifiés temporairement; l'orifice d'écoulement du tuyau (4), aussi bien en position initiale, par ex. fermé ou à demi ouvert, qu'à l'état de bolus, par ex. complètement ouvert, pouvant être détérmine à volonté par une vis de réglage (8),
**caractérisé en ce que**
- l'élément de déclenchement est un bouton de déclenchement,
- et qu'il y a un embout d'introduction (5) dans lequel le tuyau (4) est inséré
- et qu'une pince (20) en forme d'agrafe agit de l'extérieur sur le tuyau,
- l'élément de serrage est un levier de blocage (19) et la pince (20) est placée à l'extrémité du levier de blocage dont la position par rapport au tuyau peut être variée au moyen de l'élément de dosage,
- et l'élément de dosage possède en plus un moteur radial (16) sur l'axe de rotation (17) prolongé duquel le levier de blocage (19) est fixé solidement.,
- et ce moteur radial (16) est actionné électriquement et commandé électroniquement
un par une électronique de commande (1) intégrée, qui agit sur un axe de rotation (17) en même temps qu'un codeur angulaire (18), avec lequel est mesurée la position de l'axe de rotation (17) du moteur ou de la pince (20),
- et sur cette électronique de commande (1) est en outre disposé le bouton de déclenchement (11) qui, quand il est actionné, peut annuler l'action de l'élément de serrage (20).

4. Dispositif selon la revendication 3, **caractérisé en ce que** sont disposées sur l'électronique (1) des vis de réglage (8) servant à doser la quantité de médicament administré et à régler les limitations de quantités et/ou les limitations de temps d'administration du médicament.

5. Dispositif selon la revendication 3, **caractérisé en que** sur le levier de blocage (19), dans la zone de la pince (20), est placé un ressort de rappel (21) qui, en cas de fonctionnement défectueux du moteur (16) ou de la commande électronique (1), comprime ladite pince contre le tuyau 84) et bloque ainsi le flux.

6. Dispositif d'autodosage et de réglage, c-à-d de limitation et d'arrêt, du dosage de l'administration d'un médicament liquide, comprenant un contenant, un tuyau et un cathéter, pour lequel le médicament à être administer, par ex. un analgésique, se trouve dans le contenant et est introduit dans le système vas- , culaire du patient par la mise en oeuvre d'un système de perfusion ou de transfusion conventionnel, de préférence fonctionnant par gravité, sans système de transport, par ex. en forme d'une pompe supplémentaire, au moyen du tuyau et du cathéter situé à son extrémité,
le dispositif comportant un élément de dosage pouvant être réglé à volonté manuellement et doté d'un élément de déclenchement (11) et d'un élément de serrage (19),
l'élément de dosage étant disposé sur la partie extérieure du tuyau (4)
et l'élément de déclenchement (11) pouvant être actionné manuellement à volonté et étant en liaison mécanique avec un élément de serrage (10) réglable, ce qui a pour effet que l'orifice d'écoulement (la coupe transversale) du tuyau (4) et par conséquent le flux du médicament à être administré sont modifiés temporairement; l'orifice d'écoulement du tuyau (4), aussi bien en position initiale, par ex. fermé ou à demi ouvert, qu'à l'état de bolus, par ex. complètement ouvert, pouvant être détérmine à volonté,
**caractérisé en ce que**
- l'élément de déclenchement est un bouton de déclenchement,
- et qu'il y a un embout d'introduction (5), dans lequel le tuyau (4) est inséré,
- et l'élément de serrage est un axe linéaire (23)
- une pince (20) en forme d'agrafe étant placée à l'extrémité de l'axe linéaire (23) et dont la position par rapport au tuyau peut être variée au moyen de l'élément de dosage, et cette pince pouvant agir de l'extérieur sur le tuyau,
- et l'élément de dosage possède en plus un moteur linéaire ou une vanne magnétique (22), permettant de mouvoir l'axe linéaire (23) par rapport au tuyau,
- et le réglage du moteur linéaire ou de la vanne magnétique (22) et de la pression exercée sur le tuyau (4) est effectué à l'aide de l'électronique de commande (1),
- et sur cette électronique de commande (1) est en outre disposé le bouton de
déclenchement (11) qui, quand il est actionné, peut annuler l'action du levier. de blocage (20).

7. Dispositif selon la revendication 6, **caractérisé en ce que** sur l'axe linéaire (23), du côté opposé à l'élément de serrage (20), se trouve une vis de réglage de la contrainte (24) graduée, au moyen de laquelle l'élément de serrage (20) peut être détaché du tuyau (4) dans le but de régler une quantité basique de perfusion.

8. Dispositif selon la revendication 1, **caractérisé en ce** l'appareil de dosage selon la revendication 1 est combiné avec un élément de réglage ou de blocage ou d'interruption (17, 27 à 34) destiné à interrompre l'écoulement d'un médicament liquide à travers le tuyau (4) pendant une durée de temps déterminée ; l'élément de réglage ou de blocage ou d'interruption pouvant agir en combinaison avec l'élément de dosage pour interrompre l'écoulement du médicament liquide dans le tuyau (4) pendant des durées de temps déterminées,
un levier à crans (27) étant à une de ses extrémités en contact avec la surface de pression du bouton de déclenchement (11) du bouton à tapecul (3) et étant monté en rotation sur un axe (17) dans la zone de son extrémité longitudinale opposée, et qu'en outre entre cet axe (17) et l'extrémité en contact avec le bouton de déclenchement (11), le levier à crans (27) présente une partie en saillie (29) en forme d'agrafe, qui elle est appliquée par adhérence sur le bord externe d'un disque d'arrêt (30), ce disque d'arrêt (30) étant en outre situé sur un axe (31') parallèle à l'axe (17) et au tuyau (4), mu sur le même axe par un mécanisme d'horloge (31) ; le disque d'arrêt (30) ayant une ou plusieurs rainures (33) avec des lèvres (32) disposées devant, sur le bord externe du disque d'arrêt (30), et le levier à crans (27) étant garni du côté opposé au bouton de déclenchement (11) d'un ressort de pression (34) qui déclenche le bouton de déclenchement (11) à l'aide du levier à crans (27) et libère ainsi complètement le flux à travers le tuyau (4), dès que et tant que la partie en saillie (29) du levier à crans (27) glisse dans une des rainures (33) du disque d'arrêt (30) pendant la rotation dudit disque et en ce qu'enfin, un autre bouton de déclenchement (28), placé dans le boîtier (15) du dispositif et appliqué sur le disque d'arrêt (27), à son extrémité située devant l'axe (17) et opposée à la partie saillante (29), soulève par pression la partie en saillie (29) et supprime ainsi le blocage effectué par la lèvre (32) du disque d'arrêt.

9. Dispositif selon la revendication 8, **caractérisé en ce que** le levier à crans (27) est déplacé par le bouton de déclenchement (11) au-dessus de la partie en saillie (29) et du bord extérieur du disque d'arrêt (30) contre le ressort de pression (34) et est remis dans sa position initiale, après que la partie en saillie (29) a quitté une rainure (33) du disque d'arrêt et que le levier à crans (27) et le bouton de déclenchement (11) restent dans cette position pendant ce temps et que ledit bouton en particulier ne peut pas être déclenché, jusqu'à ce que le disque d'arrêt (30), en poursuivant sa rotation, se heurte par une lèvre (32) du disque d'arrêt sur la partie en saillie (29) du levier à crans (27) et le bouton de déclenchement (28) soit enfoncé.

10. Dispositif selon la revendication 8, **caractérisé en ce qu'**en modifiant la vitesse de rotation du disque d'arrêt (30) par le mécanisme d'horloge (31) ou en mettant en oeuvre des disques d'arrêt (30', 30") ayant des rainures (33) de nombre, forme et emplacement différents, ou par réglage (35) du disque d'arrêt, on peut modifier les durées de blocage de l'actionnement du bouton de déclenchement (11) et de la suppression du ralentissement du flux à travers le tuyau (4).

11. Dispositif selon l'une ou plusieurs des revendications 1 à 9, **caractérisé en ce que** le contrôle de la quantité administrée de médicament liquide est effectué par compte-gouttes, compteur de débit ou une balance, tous placés dans la zone du système de perfusion (contenant et tuyau) et/ou par mesurage de la saturation en oxygène dans le sang du patient.

12. Dispositif selon l'une ou plusieurs des revendications 1, 3 et 6, **caractérisé en ce que** le bouton de déclenchement (11) est inséré dans un anneau de protection (38) et recouvert d'un capuchon (39) doté d'un ressort de rappel.

13. Dispositif selon l'une ou plusieurs des revendications 1 à 13, **caractérisé en ce que** l'élément de dosage est doté sur son boîtier (15) d'une bande de retenue (36) comprenant une fermeture (37), avec laquelle l'élément de dosage peut être fixé sur le bras du patient, sur un pied porte-sérum ou à tout autre endroit approprié.

14. Dispositif selon l'une ou plusieurs des revendications 1 à 14, **caractérisé en ce que** dans le boîtier (15) de l'élément de dosage est placé un écran (40) affichant le nombre d'actionnements du bouton de déclenchement (11) ou toutes autres données concernant le patient et l'administration de médicaments.
